# EUROPÄISCHE PATENTSCHRIFT

(11) **EP 1 001 731 B1**
(45) Veröffentlichungstag und Bekanntmachung des Hinweises auf die Patenterteilung: **12.05.2004**
(21) Anmeldenummer: 98947327.7
(22) Anmeldetag: 21.07.1998
(51) Int. Cl.: A61K 7/00

(54) **DEKORATIVE KOSMETISCHE O/W- EMULSION OHNE EMULGATOR**
DECORATIVE COSMETIC O/W EMULSION
EMULSION HUILE DANS EAU COSMETIQUE DECORATIVE

(30) Priorität: 28.07.1997 DE 19733625
(43) Veröffentlichungstag der Anmeldung: 24.05.2000
(73) Patentinhaber: LANCASTER GROUP GmbH, 55116 Mainz (DE)
(72) Erfinder: DE CLERMONT-GALLERANDE, Helene, F-08320 Cap d'Ail (FR); ZASTROW, Leonhard, MC-98000 Monaco (MC); MARSANDE, Elisabeth, F-06240 Beausoleil (FR)
(74) Vertreter: Walter, Wolf-Jürgen
(86) Internationale Anmeldenummer: PCT/DE1998/002085
(87) Internationale Veröffentlichungsnummer: WO 1999/006010

(56) Entgegenhaltungen:
- EP-A- 0 615 741
- EP-A- 0 771 566
- WO-A-92/17159
- WO-A-94/23693
- FR-A- 2 720 934
- GB-A- 2 021 411
- US-A- 3 691 273
- US-A- 4 913 743
- US-A- 5 618 522

## Beschreibung

Die Erfindung betrifft eine dekorative kosmetische O/W-Emulsion, die eine dauerhafte Wirkung aufweist und in allen Bereichen der dekorativen Kosmetik einsetzbar ist, insbesondere bei kosmetischen Grundlagen.

Im allgemeinen werden O/W-Emulsionen für Make-up's mit anionischen oder nicht-ionischen bzw. amphoteren Emulgatoren hergestellt. Hierzu sind eine Vielzahl von Emulgatoren bekannt, wie z.B. hydrophile amphotere Block-Copolymere mit kationischen und anionischen Gruppen (US-A-5252692) und Copolymere mit Nitrilgruppen enthaltenden Monomeren (EP-A-290166). Allgemein führen derartige klassische emulgätorhaltige Emulsionen oftmals zu Irritationen der Haut, insbesondere bei häufiger oder langandauernder Anwendung.

Die US-A-4913743 betrifft die Behandlung von keratinischem Material mit einer Kombination eines anionischen und eines kationischen Polymeren, um die faserförmigen Zellstrukturen zu stabilisieren und zu stützen. Die US-A-5618522 offenbart eine O/W-Emulsion mit einer ölphase von 3000 cps und einem Verdickungsmittel, das ein Polymethacrylat-Polymeres sein kann, sowie einer wäßrigen Phase und einem Emulgator, der wenigstens in einer Phase enthalten sein muß. Die WO94/23693 betrifft eine O/W-Emulsion zur Selbstbräunung, die neben DHA, einem Sonnenschutzmittel und einem vernetzten kationischen Polymeren einen kationischen Emulgator enthält. Die EP-A-615741 beschreibt eine O/W-Emulsion, umfassend eine amphiphile Komponente sowie ein oberflächenaktives Mittel, die zusammen ein Ge1 bilden können, öl und Wasser, wobei sich die Gesamtmenge der amphiphilen Komponente und des oberflächenaktiven Mittels an der Grenzfläche der Emulsionspartikel befinden.

Der Erfindung liegt die Aufgabe zugrunde, neue dekorative O/W-Emulsionen zu entwickeln, bei denen die o.g. Nachteile nicht auftreten und die dauerhafte Produkte bilden bei gleichzeitiger ausreichender Aufnahmefähigkeit für Pigmente in die Emulsionen.

Erfindungsgemäß ist die dekorative kosmetische O/W-Emulsion dadurch gekennzeichnet, daß die Emulsion 0,1 bis 3 Gew-% eines kationischen Gels enthält, ausgewählt unter Polyquaternium-31, kationischen Gels enthält, ausgewählt unter Polyquaternium-31, Polyquaternium-16, Polyquaternium-24, Polyquaternium-7, Polyquaternium-22, Polyquaternium-39, Polyqüaternium-28, Polyquaternium-2, Polyquaternium-10, Polyquaternium-11, Polyquaternium-37 & mineral oil & PPG trideceth, PVP-dimethylaminoethylmethacrylate copolymer, Guar hydroxypropyltriammonium chloride, Calciumalginat, Ammoniumalginat, Chitosan PCA und Gemischen davon, wobei das Ge1 im Gemisch mit Wasser und mit 8 bis 20 Gew-%, bezogen auf die Gesamtmasse der Emulsion, mit dem Ge1 kompatiblen pulverförmigen Komponenten für dekorative Zwecke ein positives Zeta-Potential der Emulsion im Bereich von +5 bis +45 mV bei einem pH-Wert von 3,0 bis 8,0 ausbildet, und daß in der wäßrigen Gelphase ohne Zusatz eines Emulgators 5 bis 30 Gew-% einer ölphase stabil dispergiert sind, wobei die ölphase ausgewählt ist unter solchen Triglyceriden, Fettalkoholen, linearen und verzweigten Estern, Estern von Polyolen, pflanzlichen Ölen oder Gemischen davon, die im Gemisch mit dem kationischen Ge1 und den pulverförmigen Komponenten ein positives Zeta-Potential ergeben.

Die Emulsion kann weitere Wirkstoffe, Trägerstoffe oder Gemische davon enthalten, wobei entsprechend des isoelektrischen Punktes aller eingesetzten Rohmaterialien die Verhältnisse der Komponenten untereinander so eingestellt sind, daß sich insgesamt ein positives Zeta-Potential ergibt.

Besonders vorteilhaft ist der Einsatz von 0,3 bis 2,5 Gew-% Gelierungsmittel in der wäßrigen Phase, das zu einem positiven Zeta-Potential in der Emulsion entscheidend beiträgt. Speziell bevorzugt Gele sind Polyquaternium-10 und -37, Chitosan PCA, Guar Hydroxypropyltriammoniumchlorid allein oder als Gemisch mit einem oder mehreren davon.

Die Gelierungsmittel übernehmen zugleich die Aufgabe des Emulgators und sind daher sowohl hinsichtlich ihres eigenen Zeta-Potentials als auch hinsichtlich ihrer emulgierenden Eigenschaften speziell in dekorativen O/W-Emulsionen auszuwählen.

Durch Zusatz von ethoxyliertem Glycol oder von Polyethylenglycol (PEG) in der wäßrigen Phase kann die Teilchengröße der Emulsion weiter verringert werden. Geeignete PEG's sind z.B. Liponic EG1® von Lipo oder Lutrol e400® von BASF.

Geeignete Ester von Polyolen sind solche von C₁₀-C₁₅-Fettsäuren und Alkoholen, Ester von C₁₀-C₁₅-Fettsäuren und Glycolen oder Ester von Hydroxyfettsäuren. Besonders vorteilhaft in der ölphase sind verzweigte C₁₂-C₁₅-Alkylester in Verbindung mit anderen Estern wie Di- oder Tri-ester von Polyolen, wobei Ester von geradkettigen Alkoholen und verzweigten Säuren besonders bevorzugt sind. Alle diesen geeigneten Ester leiten sich von primären Alkoholen ab.

Zu geeigneten Stoffen für die ölphase gehören C12-15 Alkylmalate, Neopentyl Glycol Diheptanoate, Propylene Glycol Dicaprylate, Dioctyl Adipate, Diisopropyl Dimer Dilinoleate, Diisostearyl Dimer Dilinoleate, C12-13 Alkyl Lactate, Di C12-13 Alkyl Tartrate, Tri C12-13 Alkyl Citrate, C12-15 Alkyl Lactate, PPG Dioctanoate, Diethylene Glycol Dioctanoate, Meadowfoam Oil, Babassu Oil, Jojoba Oil, Rice Oil, C12-15 Alkyl Oleate, Avocado Oil, Tridecyl Neopentanoate, Beeswax, Cetearyl Alcohol and Polysorbate 60, Candellilla Wax; C18-26 Triglycerides, Cetearyl Alcohol & Cetearyl Glucoside, Acetylated Lanolin, Glyceryl Hydroxystearate; C18-36 Acid Glycol Ester, wobei solche Substanzen wie C18-36 Acid Triglycerides, Glyceryl Hydroxystearate, Candellilla Wax und Gemische davon besonders bevorzugt sind.

Es können auch solche Polymere wie Polyisobutene, Sythetic Wax, Hydrögenated Polyisobutene, Phytantriol, PVP/Hexadecene Copolymer enthalten sein.

Der Anteil der ölphase liegt vorteilhaft im Bereich von 10 bis 25 Gew-%, bezogen auf die Gesamtmasse der Emulsion. Besonders bevorzugt ist der Bereich von 15 bis 25 Gew-%.

Die erfindungsgemäße Emulsion für die dekorative Kosmetik enthält pulverförmige Substanzen, die ausgewählt sind aus der Gruppe, bestehend aus Pigmenten auf Basis von Metalloxiden (Eisenoxide, TiO₂, Ultramarinblau, Chromoxide, Glimmer-Titanoxid, Glimmer-Eisenoxid, Manganpurpur, Zinkoxid, Gemische davon) und anderer pulverförmiger Stoffe, wie SiO₂, Silica, Kaolin, mit SiO₂ modifiziertem Kaolin (gemäß WO96/17588), Polytetrafluorethylen, Nylon®, Talkum, Glimmer, Polymethylmethacrylat, Polyethylen, natürlichen organischen Verbindungen wie verkapselte oder unverkapselte Getreidestärke, Mattierungsmittel wie Polymethylmethacrylat und Gemischen davon.

Die Emulsion kann auch hohe Anteile an Aktivkomponenten enthalten, die sonst nur schwierig in einer Formulierung zu stabilisieren sind, wie Octylmethoxycinnamate, das als organisches Sonnenschutzmittel eingesetzt wird, oder Methyllactat als Erfrischungsmittel.

Die erfindungsgemäße Emulsion stellt ein kolloidales System dar.

In Abhängigkeit von dem ausgewählten kationischen Ge1 oder Gelgemisch kann die erfindungsgemäße Emulsion viskoplastisch sein, z.B. mit einem Polyquaternium-Gel wie Polyquaternium-31. Sie hat dann einen mittleren Fließpunkt (yield point) im Bereich von 15 bis 40 Pa. Die Viskosität dieser Emulsion ist kleiner als 10 Pa·s. Die Werte wurden mittels eine Rheometers vom Typ Physica US 200 nach der Herschel-Buckley-Gleichung bestimmt. Der Konus war MK23 (2°).

Ein wesentliches Merkmal der Erfindung ist das positive Zeta-Potential in der Emulsion. Das Zeta-Potential ist die Bezeichnung für die Galvanispannung im diffusen Teil der elektrochemischen Doppelschicht an der Grenzfläche zweier nicht mischbarer Phasen. Die elektrochemische Doppelschicht besteht bekanntlich aus der Stern-Schicht fest adsorbierter Ionen mit ihrem Oberflächen- oder Nernst-Potential (dem inneren Partikeln - hier: den öltröpfchen zugewandt) und dem sogenannten Stern-Potential, das der diffusen Schicht (Gony-Chapman-Doppelschicht) zugewandt ist. Das für die elektrokinetische Erscheinung der Teilchen nach außen wirksame Potential ist das Zeta-Potential. Wenn dieses Potential gleich Null ist, ist der isoelektrische Punkt erreicht.

Der pH-Wert der erfindungsgemäßen Emulsion liegt vorzugsweise im Bereich von 5,5 bis 6,5.

Es wurde nun gefunden, daß O/W-Emulsionen der oben genannten erfindungsgemäßen Zusammensetzung mit positivem Zeta-Potential eine ausgezeichnete Haftung an der Haut aufweisen und so zu sehr dauerhaften kosmetischen Produkten verarbeitet werden können, ohne daß Hautirritationen infolge Emulgatoreinwirkungen zu befürchten sind. Das ist besonders überraschend, weil aus J.Biomat.Appl., Vol. 3, April 1989, S. 554 hervorgeht, daß Hydrogele allgemein eine geringe Neigung haben, an Gewebe und ähnlichem zu haften infolge ihrer unspezifischen Absorption von Proteinen an den Hydrogel-Oberflächen, speziell wegen der fehlenden Proteinabsorption über hydrophobe Wechselwirkungen. Die sehr gute Hauthaftung ist einer der wesentlichen Vorteile der Erfindung.

Außerdem zeigen die erfindungsgemäßen Emulsionen gegenüber bekannten dekorativen Emulsionen eine erhöhte Farbstabilität, was nicht ohne weiteres aus der Zusammensetzung ableitbar war.

Es wurde weiterhin gefunden, daß die spezielle Gelphase der erfindungsgemäßen Emulsionen einen hohen Anteil an Pigmenten bzw. pulverförmigen Stoffen aufnehmen kann, und sich diese viskoplastische Emulsion daher besonders für den Einsatz in der dekorativen Kosmetik eignet. Bevorzugte Anteile an Pigmenten und/oder pulverförmigen Stoffen liegen daher bei 8-20 Gew-%, vorzugsweise 10-20 Gew-%, insbesondere 12-20 Gew-%.

Es wurde weiterhin gefunden, daß bereits geringe Mengen an kationischen Gelierungsmitteln von etwa 0,1 bis 0,8 Gew-%, die ein positives Zeta-Potential in der Emulsion haben, stabile Emulsionen bilden.

Um ein insgesamt positives Zeta-Potential im beanspruchten Bereich zu erhalten, ist es erforderlich, solche Rohmaterialien einzusetzen, die entsprechend dazu beitragen. Dazu ist es für den Fachmann notwendig, die Oberflächenladung d.h. den isoelektrischen Punkt aller eingesetzten Rohmaterialien festzustellen und die Verhältnisse der Komponenten untereinander so einzustellen, daß sich insgesamt ein positives Zeta-Potential ergibt.

So setzen beispielsweise Pigmente das Zeta-Potential etwas herab, da mineralische Substanzen bei den pH-Werten der erfindungsgemäßen Emulsion, die im Bereich von 5,5-6,5 liegen, oft negativ geladen sind, dieser pH-Wert aber meist in Nähe des isoelektrischen Punktes der Pigmente liegt. Es sind daher vom Fachmann geeignete kationische Gele auszuwählen, um zusammen mit den weiteren Wirkstoffen und/oder Trägerstoffen ein insgesamt posivites Zeta-Potential zu erreichen. Dabei können strukturelle Ähnlichkeiten zwischen z.B Polyquaternium-31 (quaternäres Ammoniumsalz aus DMAPA Acrylates/Acrylic Acid/Acrylonitrogene Copolymer und Diethylsulfat) und einem Acrylat-Blockcopolymeren durchaus völlig andere Zeta-Potentiale in der Emulsion hervorrufen, z.B. +38 mV mit Polyquaternium-31 und -21 mV mit einem Acrylat-Blockcopolymeren.

Schließlich wurde auch gefunden, daß die Feuchtigkeitseigenschaften der erfindungsgemäßen Emulsion etwas besser sind als die von vergleichbaren Emulsionen, die zusätzliche Feuchthaltemittel enthalten. So zeigen Corneometer®-Messungen der Hauthydratation 1 Stunde nach Auftragen der Emulsion einen Wert von 12 % gegenüber 11 % der Vergleichsemulsion, die Feuchthaltemittel enthielt. Nach 3 Stunden zeigte die erfindungsgemäße Emulsion einen Wert von 4,5 % gegenüber 2 % der Vergleichsemulsion. Die Vergleichsemulsion war eine sogenannte "no-transfer Foundation". Signifikant noch bessere Ergebnisse werden im Vergleich mit einer sogenannten "transferproof Foundation" erwartet. Von derartigen Emulsionen ist bekannt, daß sie dehydratisierende Kosmetika sind.

Die besseren Feuchtigkeitseigenschaften der erfindungsgemäßen Emulsion sind wahrscheinlich auf die Kapazität des Hydrogels zurückzuführen, Wasser über einen langen Zeitraum in seinem Netzwerk zu halten.

Die durch die Erfindung zu erreichende besonders günstige langandauernde Haftung läßt sich nachweisen durch Untersuchung von z.B. der Farbänderung ΔE in einer mit Pigmenten versetzten erfindungsgemäßen Formulierung, die auf eine Gesichtshälfte eines Probanden aufgetragen wurde, im Vergleich mit einer Formulierung gleicher Zusammensetzung, jedoch mit negativem Zeta-Potential der Emulsion. Während bei einer Formulierung mit negativem Zeta-Potential der ΔE-Wert von etwa 22 acht Stunden nach dem Auftragen auf etwa 18 abfällt, bleibt der ΔE-Wert der auf der Gesichtshälfte aufgetragenen Emulsion der Erfindung mit positivem Zeta-Potential über den gesamten Zeitraum nahezu konstant beim Ausgangswert. Merkmal einer langandauernden Wirkung, wie sie für die vorliegende Erfindung charakteristisch ist, ist somit die Haftung an der Haut ohne merkliche farbliche oder sonstige Beeinträchtigung des Zustandes der auf die Haut aufgetragenen Emulsion über einen Zeitraum von wenigstens 3 Stunden.

Eine spezielle Ausführungsform der Erfindung besteht in einer kosmetischen Emulsion, bei der die wäßrige Phase 0,1 bis 0,8 Gew-% eines kationischen Gels oder Gelgemisches enthält, das im Gemisch mit Wasser und mit 0,1 bis 20 Gew-% pulverförmigen Komponenten und gegebenenfalls anderen Wirkstoffen, Trägerstoffen oder Gemischen davon ein positives Zeta-Potential der Emulsion im Bereich von +5 bis +45 mV bei einem pH-Wert von 3,0 bis 8,0, vorzugsweise 5,5 bis 6,5 ausbildet, und daß in der wäßrigen Gelphase ohne Zusatz eines Emulgators 5 bis 30 Gew-% einer ölphase stabil dispergiert sind.

Die Erfindung soll nachstehend durch Beispiele näher erläutert werden. Alle Angaben der Komponenten sind in Gewichts-%.

### Beispiel 1 Emulgatorfreie Emulsion (nicht erfindungsgemäß)

| | |
|---|---|
| Deionisiertes Wasser | q.s. ad 100 |
| Konservierungsmittel | 0,5 |
| Glycereth-26 oder PEG8 | 4,0 |
| Polyquaternium-10 | 0,5 |
| Propylene Glycol Dicaprilate Dicaprate | 0,3 |
| PPG-1-Trideceth-6 | 0,7 |
| Tocopheryl Acetate | 0,2 |
| C₁₂-C₁₃-alkyl Malate | 25,0 |

Die Herstellung der Emulsion erfolgte in üblicher Weise, indem das Wasser auf 80 °C unter Rühren erwärmt wurde und Polyquaternium-10 unter Homogenisierung bei etwa 8000 U/Min. zugegeben wurde. Die ölphase wurde auf 80 °C erwärmt, und die Emulgierung mit der wäßrigen Phase erfolgte bei 8000 U/Min. Danach wurde das Produkt unter langsamem Rühren (etwa 200 U/Min) entlüftet und abgekühlt. Die erhaltene Emulsion hatte ein Zeta-Potential von +28 mV bei einem pH-Wert von 6 und eine volumenbezogene mittlere Teilchengröße von 2,56 µm, wobei 90 % der Teilchen kleiner als 6,33 µm waren.

Messungen des Zeta-Potentials erfolgten auf einem "Zeta Sizer 3" von Malvern Instruments Ltd., Worcs, Großbritannien. Das Zeta-Potential wurde dabei auf Basis eine Laser-Doppler-Mikroelektrophorese ermittelt. Dabei wird die Emulsion verdünnt in eine Meßzelle injiziert, ein Strom mit einer bekannten Spannung angelegt und die Messung der Geschwindigkeit der Teilchen in einem Flüssigkeitsstrom über Laser-Doppler-Velocimetrie (LDV) vorgenommen und in Abhängigkeit von der Viskosität, der Wanderungsgeschwindigkeit im Gleichstromfeld, der Dielektrizitätskonstante und der Feldstärke nach der bekannten Formel ermittelt.

Die Messungen der Teilchengrößen erfolgten auf einem "Mastersizer" von Malvern Instruments Ltd.

### Beispiel 2 Kosmetische Grundlage mit Sonnenschutz (nicht erfindungsgemäß)

| | |
|---|---|
| Deionisiertes Wasser | q.s. ad 100 |
| Konservierungsmittel | 0,5 |
| PEG - 8 oder Glycereth -2S | 4,0 |
| Polyquaternium-10 | 0,4 |
| Eisenoxide | 1,25 |
| Titanium Dioxide | 5,0 |
| Tocopheryl Acetate | 0,2 |
| C₁₂-C₁₃-alkyl Octanoate | 18,0 |
| Octylmethoxycinnamate | 7,0 |

Die Herstellung der Emulsion erfolgte in ähnlicher Weise wie im Beispiel 1, jedoch wurden die Pigmente in Glycereth-25 oder PEG 8 vor der Zugabe der wäßrigen Phase hinzugesetzt. Octylmethoxycinnamate wurde während der Abkühlphase bei etwa 60 °C hinzugegeben.

Die erhaltene Emulsion hatte in vitro einen Sonnenschutzfaktor (SPF) von 10 und ein Zeta-Potential von +35,8 mV ±1,2 mV, wobei 80 % der Teilchen eine Teilchengröße von weniger als 8,80 µm hatten.

### Beispiel 3 Kosmetische Grundlage

| | |
|---|---|
| Deionisiertes Wasser | q.s. ad 100 |
| Konservierungsmittel | 0,5 |
| Kaolin modifiziert mit SiO₂* | 2,5 |
| Polyquaternium-10 | 0,4 |
| Chitosan PCA | 1,5 |
| Glycereth-26 | 4,0 |
| Silica | 1,0 |
| Di-C₁₂-C₁₃ Alkyl Malate | 11,0 |
| Eisenoxide | 1,25 |
| Titaniumdioxid | 5,0 |
| Neopentyl Glycol Diheptanoate | 3,0 |
| Diethylene Glycol Dioctanoate/ Diisononanoate | 3,5 |
| Tridecyl Neopentanoate | 2,0 |
| Tocopheryl Acetate | 0,2 |
| Myristyl Lactate | 2,0 |
| Cyclomethicone & Dimethyconol | 1,0 |
| Porphyridium Cruentum Extract | 5,0 |
| Parfümöl | 0,4 |

Die Herstellung der Emulsion erfolgte in ähnlicher Weise wie im Beispiel 2. Die erhaltene Emulsion hatte ein Zeta-Potential von +32,4 ±1,5 mV bei einem pH-Wert von 6.
* Weißer Kaolin modifiziert gemäß WO96/17588 mit 0,5 - 10 Gew-% sphärischem SiO₂ mit einer Teilchengröße <5 µm.

### Beispiel 4 Wimperntusche (nicht zur Erfindung gehörig)

| | |
|---|---|
| Deionisiertes Wasser | q.s. ad 100 |
| Konservierungsmittel | 0,7 |
| PVP | 4,0 |
| Polyquaternium-10 | 0,2 |
| Guar Hydroxypropyltriammoniumchlorid | 0,9 |
| Glycereth 26 | 2,0 |
| Triethanolamine 99% | 2,4 |
| Magnesium Aluminium Silicate | 1,0 |
| Talkum | 1,0 |
| Weizenkeimöl | 1,0 |
| PVP/Eicosene Copolymer | 2,0 |
| Eisenoxide | 12,0 |
| Hydrogenated Polyisobutene | 0,2 |
| Cetearyl Alcohol | 0,1 |
| Stearinsäure | 7,0 |
| Carnauba | 4,0 |
| Sorbitan Sesquioleate | 1,3 |
| Bienenwachs | 4,0 |
| C18-36 Acid Triglycerides | 8,5 |
| Lecithin | 1,0 |
| Tocopheryl Acetate | 0,2 |

Die Gele und Polymere wurden alle in der wäßrigen Phase dispergiert. Anschließend wurden die Pigmente dispergiert. Die Fertigstellung der Emulsion erfolgte bei 85 °C.

Die erhaltene Emulsion hatte ein Zeta-Potential von 29,1 mV ±1,6 mV. Wegen der hohen Wachskonzentrationen ist zur Stabilisierung ein Emulgator enthalten.

### Beispiel 5 Eyeliner

| | |
|---|---|
| Deionisiertes Wasser | q.s. ad 100 |
| Konservierungsmittel | 0,5 |
| PVP | 1,0 |
| Polyquaternium-37 | 0,5 |
| Chitosan PCA | 0,5 |
| Propylene Glycol Dicaprilate Dicaprate | 0,3 |
| PPG-1-Trideceth-6 | 0,7 |
| Glycereth 26 oder PEG 8 | 5,0 |
| Eisenoxide | 10,0 |
| Propylene Glycol Hydroxystearate | 11,5 |
| Hydrogenated Polyisobutene | 3,0 |
| Cyclomethicone & Dimethyconol | 2,0 |
| Tocopheryl Acetate | 0,5 |

Die Herstellung der Emulsion erfolgte in ähnlicher Weise wie im Beispiel 2. Die erhaltene Emulsion hatte ein Zeta-Potential von +18,1 ±4,5 mV.

### Beispiel 6 Kosmetische Grundierung (Foundation)

Diese kosmetische Grundierung wird vorteilhaft nicht auf Textilien übertragen.

| | |
|---|---|
| Deionisiertes Wasser | q.s. ad 100 |
| Konservierungsstoffe | 0,5 |
| Glycereth 26 | 4,0 |
| C₁₂-C₁₃ Alkyl Malate | 4,2 |
| Eisenoxide | 10,5 |
| Polyquaternium-31 | 0,4 |
| Diethylene Glycol Dioctanoate/ Diisononanoate | 4,5 |
| Tocopheryl Acetate | 0,2 |
| Myristyl Lactate | 3,5 |
| Dicaprylyl Ether | 3,0 |
| Cetearyl Alcohol | 4,0 |
| Jojoba-ester | 1,5 |
| Polymethyl Methacrylate | 2,5 |
| Parfümöl | 0,4 |

Die Herstellung der Emulsion erfolgte in ähnlicher Weise wie im Beispiel 2. Die erhaltene Emulsion hatte ein Zeta-Potential von +35,8 ±1,2 mV für einen pH-Wert von 6.

### Vergleichsbeispiel 1

Es wurde eine ähnliche Emulsion wie im Beispiel 3 hergestellt mit der folgenden Zusammensetzung

| | |
|---|---|
| Deionisiertes Wasser | q.s. ad 100 |
| Konservierungsmittel | 0,5 |
| Kaolin modifiziert mit SiO₂* | 1,0 |
| Xanthangummi | 0,3 |
| Silica | 1,0 |
| Di-C₁₂-C₁₃ Alkyl Malate | 11,0 |
| Propylenglycol | 6,25 |
| Eisenoxide | 1,25 |
| Titaniumdioxid | 5,0 |
| Neopentyl Glycol Diheptanoate | 3,0 |
| Diethylene Glycol Dioctanoate/ Diisononanoate | 3,5 |
| Tridecyl Neopentanoate | 2,0 |
| Acrylic Acid/Acrylonitrogens Copolymer | 3,4 |
| Myristyl Lactate | 2,0 |
| Cyclomethicone & Dimethyconol | 1,0 |
| Parfümöl | 0,4 |

| | |
|---|---|
| * gemäß WO96/17588 | |

Die Herstellung der Emulsion erfolgte in ähnlicher Weise wie im Beispiel 3. Die erhaltene Emulsion hatte ein Zeta-Potential von -41,4 ±7,9 mV für einen pH-Wert von 5,5.

Die Emulsion von Beispiel 3 und vom Vergleichsbeispiel 1 wurde einem Farbtest zur Feststellung von ΔE über einen Zeitraum 8 Stunden unterworfen. Man erhielt die bereits weiter oben angegebenen Ergebnisse.

## Patentansprüche

1. Dekorative kosmetische O/W-Emulsion, **dadurch gekennzeichnet, daß** die Emulsion 0,1 bis 3 Gew-% eines kationischen Gels enthält, ausgewählt unter Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium-37 & mineral oil & PPG trideceth, PVP-dimethylaminoethylmethacrylate copolymer, Guar hydroxypropyltriammonium chloride, Calciumalginat, Ammoniumalginat, Chitosan PCA und Gemischen davon, wobei das Gel im Gemisch mit Wasser und mit 8 bis 20 Gew-%, bezogen auf die Gesamtmasse der Emulsion, mit dem Gel kompatiblen pulverförmigen Komponenten für dekorative Zwecke ein positives Zeta-Potential der Emulsion im Bereich von +5 bis +45 mV bei einem pH-Wert von 3,0 bis 8,0 ausbildet, und
daß in der wäßrigen Gelphase ohne Zusatz eines Emulgators 5 bis 30 Gew-% einer ölphase stabil dispergiert sind, wobei die ölphase ausgewählt ist unter solchen Triglyceriden, Fettalkoholen, linearen und verzweigten Estern, Estern von Polyolen, pflanzlichen ölen oder Gemischen davon, die im Gemisch mit dem kationischen Gel und den pulverförmigen Komponenten ein positives Zeta-Potential ergeben,
und wobei die Messung des Zeta-Potentials durch Messung der Geschwindigkeit der Emulsionsteilchen bei bekannter Spannung in einem Flüssigkeitsstrom über Laser-Doppler-Velocimetrie erfolgt und der Wert in Abhängigkeit von der Viskosität, der Wanderungsgeschwindigkeit im Gleichstromfeld, der Dielektrizitätskonstante und der Feldstärke ermittelt wird.

2. Kosmetische Emulsion nach Anspruch 1, **dadurch gekennzeichnet, daß** sie weitere Wirkstoffe, Trägerstoffen oder Gemischen davon enthält, wobei entsprechend des isoelektrischen Punktes aller eingesetzten Rohmaterialien die Verhältnisse der Komponenten untereinander so eingestellt sind, daß sich insgesamt ein positives Zeta-Potential ergibt.

3. Kosmetische Emulsion nach Anspruch 1, **dadurch gekennzeichnet, daß** die wäßrige Phase ein Gelierungsmittel in einem Anteil von 0,1 bis 0,8 Gew-% in der wäßrigen Phase enthält, das zu einem positivem Zeta-Potential in der Emulsion führt.

4. Kosmetische Emulsion nach Anspruch 1, **dadurch gekennzeichnet, daß** das Gelierungsmittel Polyquaternium 10, Polyquaternium 37, Guar Hydroxypropyltriammoniumchlorid oder ein Gemisch davon ist.

5. Kosmetische Emulsion nach Anspruch 1 oder 4, **dadurch gekennzeichnet, daß** das Gelierungsmittel Chitosan PCA oder ein Gemisch mit einem der Gele Polyquaternium 10, Polyquaternium 37 oder Guar Hydroxypropyltriammoniumchlorid ist.

6. Kosmetische Emulsion nach Anspruch 1, **dadurch gekennzeichnet, daß** die Triglyceride der Ölphase C18-36 Acid Triglycerides, Glyceryl Hydroxystearate, Candellilla Wax und Gemische davon umfassen.

7. Kosmetische Emulsion nach Anspruch 1, **dadurch gekennzeichnet, daß** die Fettalkohole Cetearyl Alcohol und Stearyl Alcohol umfassen.

8. Kosmetische Emulsion nach einem der Ansprüche 1 bis 3, **dadurch gekennzeichnet, daß** die wäßrige Phase zusätzlich ein Polyethylenglycol enthält.

9. Kosmetische Emulsion nach Anspruch 1, **dadurch gekennzeichnet, daß** die wäßrige Phase pulverförmigen Komponenten enthält, die aus der Gruppe ausgewählt sind, bestehend aus Pigmenten auf Basis von Metalloxiden, wie Eisenoxide, TiO₂, Ultramarinblau, Glimmertitanoxid, Glimmer-Eisenoxide, Zinkoxid, Gemische davon; anderen pulverförmigen Stoffe, wie Silica, Kaolin, mit SiO₂ modifizierter Kaolin, Polytetrafluorethylen, Nylon®, Talkum, Glimmer, Polyethylen, natürliche organische Verbindungen wie verkapselte oder unverkapselte Getreidestärke; und Gemische davon.

10. Kosmetische Emulsion nach einem der Ansprüche 1 bis 9, **dadurch gekennzeichnet, daß** die Emulsion eine Teilchengröße von kleiner als 15 µm hat.

11. Kosmetische Emulsion nach einem der Ansprüche 1 bis 10, **dadurch gekennzeichnet, daß** der Anteil der ölphase im Bereich von 10 bis 20 Gew-% liegt, bezogen auf die Gesamtmasse der Emulsion.

12. Kosmetische Emulsion nach Anspruch 1, **dadurch gekennzeichnet, daß** der pH-Wert der Emulsion im Bereich von 5,5-6,5 liegt.

13. Kosmetische Emulsion nach einem der Ansprüche 1 bis 5, **dadurch gekennzeichnet, daß** die wäßrige Phase 0,1 bis 0,8 Gew-% eines kationischen Gels oder Gelgemisches enthält.

## Claims

1. Decorative cosmetic O/W emulsion, which comprises 0.1 to 0.3 % by weight of a cationic gel selected among polyquaternium-31, polyquaternium-16, polyquaternium-24, polyquaternium-7, polyquaternium-22, polyquaternium-39, polyquaternium-28, polyquaternium-2, polyquaternium-10, polyquaternium-11, polyquaternium-37 & mineral oil & PPG trideceth, PVP dimethylaminoethylmethacrylate copolymer, Guar hydroxypropyltriammonium chloride, calcium alginate, ammonium alginate, Chitosan PCA and mixtures thereof, wherein the gel in a mixture with water and, related to the total amount of the emulsion, with 8 to 20 % by weight of gel-compatible powdery components for decorative purposes provides a positive zeta potential of the emulsion in the range of +5 to +45 mV at a pH value of 3.0 to 8.0, and
in that 5 to 30 % by weight of an oil phase are dispersed in a stable manner in the aqueous gel phase without adding an emulsifier, with the oil phase being selected among those triglycerides, fatty alcohols, linear and branched esters, esters of polyhydroxy alcohols, vegetable oils or mixtures thereof providing in a mixture with the cationic gel and the powdery components a positive zeta potential,
and in that the zeta potential is measured by measuring the velocity of the emulsion particles at a known voltage in a liquid flow via laser Doppler velocimetry and by determining the value depending upon viscosity, the travel speed in the direct current field, the dielectric constant and the field intensity.

2. Cosmetic emulsion according to Claim 1, wherein it contains further agents, carrier substances or mixtures thereof wherein, depending upon the isoelectric point of all raw substances used, the component ratios are adapted towards each other in a way to provide an overall positive zeta potential.

3. Cosmetic emulsion according to Claim 2, wherein the aqueous phase contains a gelatinising agent at a ratio of 0.1 to 0.8 % by weight in the aqueous phase which results in a positive zeta potential in the emulsion.

4. Cosmetic emulsion according to Claim 1, wherein the gelatinising agent is polyquaternium-10, polyquaternium-37, Guar hydroxypropyltriammonium chloride or a mixture thereof.

5. Cosmetic emulsion according to Claim 1 or Claim 4, wherein the gelatinising agent is Chitosan PCA or a mixture with one of the gels polyquaternium-10, polyquaternium-37 or Guar hydroxypropyltriammonium chloride.

6. Cosmetic emulsion according to Claim 6, wherein said triglycerides comprise C₁₈₋₃₆ acid triglycerides, glyceryl hydroxystearate, candelilla wax and mixtures thereof.

7. Cosmetic emulsion according to Claim 6, wherein said fatty alcohols comprise cetearyl alcohol and stearyl alcohol.

8. Cosmetic emulsion according to any one of claims 1 to 3, wherein the aqueous phase further contains a polyethylene glycol.

9. Cosmetic emulsion according to Claim 1, wherein the aqueous phase contains powdery substances selected from the group consisting of pigments on the basis of metal oxides such as iron oxides, TiO₂, ultramarine blue, micaceous titanium oxide, micaceous iron oxides zinc oxide and mixtures thereof; other powdery substances such as silica, kaolin, kaolin modified with SiO₂, polytetrafluoroethylene, nylon^{□}, talcum, mica, polyethylene, natural organic compounds such as encapsulated or non-encapsulated grain starch; and mixtures thereof.

10. Cosmetic emulsion according to any one of claims 1 to 9, wherein the particle size in the emulsion is smaller than 15 µm.

11. Cosmetic emulsion according to claims 1 to 10, wherein the share of the oil phase is in the range of 10 to 20 % by weight, based on the total amount of the emulsion.

12. Cosmetic emulsion according to Claim 1, wherein the pH value of the emulsion is in the range from 5.5 to 6.5.

13. Cosmetic emulsion according to any of Claims 1 to 5, wherein the aqueous phase contains 0.1 to 0.8 % by weight of a cationic gel or gel mixture.

## Revendications

1. Emulsion cosmétique décorative huile/eau, **caractérisée en ce que** l'émulsion contient 0,1 à 3 % en poids d'un gel cationique, choisi parmi le polyquaternium 31 le polyquaternium 16, le polyquaternium 24, le polyquaternium 7, le polyquaterniuni 22, le polyquaternium 39, le polyquaternium 28, le polyquaternium 2, le polyquaternium 10, le polyquaternium 11, le polyquaternium 37 & mineral oil & PPG trideceth, le copolymère de PVP et de méthacrylate de diméthyaminoéthyle, le chlorure de guar hydroxypropyltriammonium, l'alginate de calcium, l'alginate d'ammonium, le chitosan PCA et des mélanges de ces derniers, sachant que le gel en mélange avec l'eau et avec 8 à 20 % en poids, rapportés à la masse totale de l'émulsion, avec des composés pulvérulents compatibles avec le gel pour des fins décoratives, développe à un pH de 3,0 à 8,0 un potentiel zêta positif de l'émulsion dans l'intervalle de +5 à +45 mV et
que sans addition d'un émulsionnant, 5 à 30 % en poids d'une phase d'huile sont dispersés de façon stable dans la phase aqueuse du gel, sachant que la phase d'huile est choisie entre ceux des triglycérides, des alcools gras, des esters linéaires et ramifiés, des esters de polyols, des huiles végétales ou des mélanges de ces derniers, qui donnent en mélange avec le gel cationique et les composants pulvérulents un potentiel zêta positif,
et sachant que le potentiel zêta a été déterminé par mesure de la vitesse des particules d'émulsion dans un courant de liquide, sous une tension connue, par vélocimétrie Doppler au laser et que la valeur est déterminée en fonction de la viscosité, de la vitesse de migration dans le champ de courant continu, de la constante diélectrique et de l'intensité du champ.

2. Emulsion cosmétique selon la revendication 1, **caractérisée en ce qu'**elle contient d'autres substances actives, des substances porteuses ou des mélanges de ces dernières, pour lesquels, en fonction du point isoélectrique de tous les matériaux bruts utilisé, les rapports des composants les uns par rapport aux autres sont réglés de telle sorte qu'il résulte en tout un potentiel zêta positif.

3. Emulsion cosmétique selon la revendication 1, **caractérisée en ce que** la phase liquide contient un agent gélifiant dans une proportion de 0,1 à 0,8 % en poids dans la phase liquide, qui conduit à un potentiel zêta positif dans l'émulsion.

4. Emulsion cosmétique selon la revendication 1, **caractérisée en ce que** l'agent gélifiant est du polyquaternium 10, du polyquaternium 37, du chlorure de guar hydroxypropyltriammonium ou un mélange de ces derniers.

5. Emulsion cosmétique selon la revendication 1 ou 4, **caractérisée en ce que** l'agent gélifiant est du chitosan PCA ou un mélange avec un des gels polyquaternium 10, polyquaternium 37 ou chlorure de guar hydroxypropyltriammonium.

6. Emulsion cosmétique selon la revendication 1, **caractérisée en ce que** les triglycérides de la phase huileuse sont des triglycérides d'acides en C₁₈ à C₃₆, de l'hydroxystéarate de glycéryle, de la cire de candellila ou des mélanges de ces derniers.

7. Emulsion cosmétique selon la revendication 1, **caractérisée en ce que** les alcools gras comprennent l'alcool cétéarylique et l'alcool stéarique.

8. Emulsion cosmétique selon l'une quelconque des revendications 1 à 3, **caractérisée en ce que** la phase aqueuse contient en plus un polyéthylène glycol.

9. Emulsion cosmétique selon la revendication 1, **caractérisée en ce que** la phase aqueuse contient des composants pulvérulents qui sont choisis dans le groupe qui consiste en des pigments à base d'oxydes métalliques, tels les oxydes de fer, le TiO₂, le bleu outremer, l'oxyde de titane micacé, les oxydes de fer micacés, l'oxyde de zinc, des mélanges de ces derniers ; d'autres substances pulvérulentes telles la silice, le kaolin, le kaolin modifié par SiO₂, le polytétrafluoréthylène, le nylon®, le talc, le mica, le polyéthylène, des composés organiques tels l'amidon de céréales encapsulé ou non encapsulé ; et des mélanges de ces derniers.

10. Emulsion cosmétique l'une quelconque des revendications 1 à 9, **caractérisée en ce que** l'émulsion a une taille de particules de moins de 15 µm.

11. Emulsion cosmétique selon l'une quelconque des revendications 1 à 10, **caractérisée en ce que** la proportion de la phase huileuse est comprise dans l'intervalle de 10 à 20 % en poids, rapportés à la masse totale de l'émulsion.

12. Emulsion cosmétique selon la revendication 1, **caractérisée en ce que** le pH de l'émulsion est compris dans l'intervalle de 5,5 à 6,5.

13. Emulsion cosmétique selon l'une quelconque des revendications 1 à 5, **caractérisée en ce que** la phase aqueuse contient 0,1 à 0,8 % en poids d'un gel cationique ou d'un mélange de gels cationiques.
